(19) Europäisches Patentamt

European Patent Office

Office européen des brevets

(11) **EP 4 368 607 B1**

(12) **EUROPEAN PATENT SPECIFICATION**

(45) Date of publication and mention
of the grant of the patent:
**18.06.2025   Bulletin 2025/25**

(21) Application number: **23207544.0**

(22) Date of filing: **02.11.2023**

(51) International Patent Classification (IPC):
*C07C 51/09* (2006.01)   *C07C 51/44* (2006.01)
*C07C 51/48* (2006.01)   *C07C 53/08* (2006.01)
*C07C 67/54* (2006.01)   *C07C 67/58* (2006.01)
*C07C 69/80* (2006.01)   *C08L 1/12* (2006.01)

(52) Cooperative Patent Classification (CPC):
(C-Sets available)
**C07C 51/09; C07C 51/44; C07C 51/48;
C07C 67/54; C07C 67/58; C08L 1/12**          (Cont.)

(54) **PROCESS FOR RECOVERING ACETIC ACID AND OTHER VALUABLE MATERIALS FROM CELLULOSE ACETATE WASTE BY MEANS OF HYDROTHERMAL LIQUEFACTION**

VERFAHREN ZUR GEWINNUNG VON ESSIGSÄURE UND ANDEREN WERTSTOFFEN AUS CELLULOSEACETATABFÄLLEN MITTELS HYDROTHERMALER VERFLÜSSIGUNG

PROCÉDÉ DE RÉCUPÉRATION D'ACIDE ACÉTIQUE ET D'AUTRES MATIÈRES VALORISABLES À PARTIR DE DÉCHETS D'ACÉTATE DE CELLULOSE PAR LIQUÉFACTION HYDROTHERMALE

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority:  **11.11.2022   IT 202200023355**

(43) Date of publication of application:
**15.05.2024   Bulletin 2024/20**

(73) Proprietors:
• **Acetek Momentum Co., Ltd.**
  **Zaozhuang City Shandong (CN)**
• **Jinan Acetate Chemical Co., Ltd.**
  **Jinan City Shandong (CN)**

(72) Inventors:
• **De Filippis, Paolo**
  **Rome (IT)**
• **De Caprariis, Benedetta**
  **Rome (IT)**

(74) Representative: **Sarpi, Maurizio et al
Studio Ferrario S.r.l.
Via Collina, 36
00187 Roma (IT)**

(56) References cited:
**CN-A- 107 673 962      CN-A- 112 047 827**

**(Cont. next page)**

(52) Cooperative Patent Classification (CPC): (Cont.)

C-Sets
**C07C 51/09, C07C 53/08;**
**C07C 51/44, C07C 53/08;**
**C07C 51/48, C07C 53/08;**
**C07C 67/54, C07C 69/80;**
**C07C 67/58, C07C 69/80**

**Description**

The present invention relates to an innovative process for recovering acetic acid useful for the production of acetic anhydride and other valuable materials from cellulose acetate waste and scrap by means of hydrothermal liquefaction.

Cellulose acetate (CA) is an environmentally friendly product from production to degradation processes. Like all other cellulose derivatives, the synthesis thereof is based on the replacement of the cellulose hydroxyl groups with other functional groups which in this case are the acetyl group.

Although three processes exist for producing cellulose acetate (the process with acetic anhydride, the process with methylene chloride, and the heterogeneous process), today only the acetic anhydride process is used, which uses cellulose as a raw material, which is nature's most abundant renewable source.

CN112047827 A discloses a method for obtaining acetic acid from waste cellulose acetate slurry, which comprises an acid degradation treatment in the presence of sulfuric acid at 90-100°C. Then, acetic acid is extracted with isopropyl acetate and separated by distillation.

SCOPE OF THE INVENTION

The recovery of cellulose acetate waste and scrap assumes a primary economic value, especially in the production of eyeglasses.

It is known that eyeglasses have become a fashion accessory in recent decades which, in addition to responding to the need to accommodate a pair of corrective sun lenses, have taken on an aesthetic value. For this reason, numerous fashion brands are present on the distribution market worldwide, reaffirming the brand concept and the perceived value of a pair of eyeglasses.

Plastic eyeglasses are better suited to the role of eyeglasses as an expression of fashion as, in addition to the model, plastic creates games of colors and structures through the three-dimensionality thereof.

When mention is made of plastic, reference is mostly made to eyeglasses milled from a sheet of cellulose acetate, a polymer of natural origin which is synthesized from cotton or wood pulp with the addition of acetic acid. The production of eyeglasses from sheets includes cutting a rectangle (170x70mm) from a sheet of different sizes (600x1400m; 170x1400mm; 170x700mm). The thickness of the sheet varies from 5 to 8 m.

The weight of the eyeglasses can be about 20 g, while the rectangle can weigh from 92 g to 122 g, with waste therefore ranging from 78% to 84%. The eyeglasses production process, although virtuous from the "aesthetic result" point of view, is flawed from the point of view of "efficiency," as it registers significant scraps in sheets which go partly to landfill and are partly used as a by-product (in the form of granules obtained from the extrusion of the waste) for items without performance expectations.

To this must be added the waste of the acetate sheet production according to the extrusion or solvent manufacturing process. Part of this waste is reused in the sheet manufacturing process itself and part is destined for the production of semi-finished products for products without performance expectations.

The sheet production centers are in Italy for about 3,000 tons/year and China for about 16,000 tons/year and the eyeglasses production centers are also located in Italy and China with some exceptions for some productions of little importance in Europe.

Considering the sheet production waste and eyeglasses production scraps together, we can estimate the European volume equal to about 2,500 tons/year, while in China mention can be made of about 14,000 tons/year.

Not using this waste and these scraps means resorting to the synthesis of new volumes of polymer (based on cellulose and acetic anhydride) and plasticizer necessary for processing, by means of extrusion and solvent, the cellulose acetate plates. This results in a consumption of new polymers of about 16,500 tons/year of polymer and 15,000 tons/ year of cellulose acetate and 6,200 tons/year of diethyl phthalate.

Currently, the only way to recover the waste and scraps from the sector is to gasify the waste, with the production of primary elements such as carbon monoxide, hydrogen, carbon dioxide and water.

Hence the need for and value of a process such as that which is the object of the present invention that instead allows decomposing cellulose acetate so as to chemically recover valuable components such as acetic acid, cellulose (even in degraded form) and diethyl phthalate used as a plasticizer.

The use of acetic anhydride for the production of cellulose acetate dates back to the 1930s with studies on synthetic-artificial plastic materials, what we commonly call "plastic" today. It was discovered then that by reacting cellulose not with nitric acid, but with acetic anhydride, a new material is obtained: cellulose acetate. It is non-flammable and the production process is simple and fast. It can perfectly replace the use of celluloid, so much so that it is still among the most used materials today.

The features of cellulose acetate depend on the average degree of substitution (DS) or the amount of acetic acid in terms of percentage "acetic acid %". The conversion of DS can be calculated according to the following equation:

$$DS = \frac{162 \ x \ \% \ Acetyl}{(43 \ x \ 100) - [ \ (43 - 1) \ x \ \% \ Acetyl]}$$

where:

- 162 is the molar weight of the cellulose monomer;
- 43 is the molar weight of the acetyl group;
- % Acetyl is the acetyl content (wt.%).

The relationship between cellulose acetate DS and acetyl content is also reported in the following Table 1.

TABLE 1

Relationship between cellulose acetate DS and combined acetyl and acetic acid content

| Compound | DS | Acetyl Content | Content Acetic Acid |
|---|---|---|---|
| Cellulose | 0 | 0.0% | |
| Cellulose Monoacetate | 1 | 21.1% | 29.4% |
| Cellulose Diacetate (CDA) | 2 | 34.9% | 48.8% |
| Cellulose Triacetate (CTA) | 3 | 44.8% | 62.5% |

For commercial applications, the cellulose acetate DS is always greater than 2 and typically 2.45. Cellulose acetate needs to be plasticized to be used as a polymer so as to lower the melting temperature thereof, which in the absence of the plasticizer is too close to the decomposition temperature thereof. The use of the plasticizer makes the polymer more flexible, durable and workable, lowering the transition temperature of the macromolecule.

Traditional cellulose acetate plasticizers are triacetin (TA) and diethyl phthalate (DEP). Diethyl phthalate is one of the most common plasticizers of cellulose acetate but, due to some toxicity/safety issues, its industrial use will be reduced in the near future and replaced by the more ecological triacetin plasticizers. A significant amount of plasticizer is generally used (between 25 and 30% by weight depending on the type of use).

The task which the inventors of the present application set themselves was to experimentally evaluate the possibility of chemically recycling cellulose acetate so as to recover valuable compounds such as acetic acid (intermediate for the production of acetic anhydride used in the synthesis of diacetate (DA), cellulose (also in degraded form) and diethyl phthalate that is used as plasticizer.

CELLULOSE ACETATE DEACETYLATION EXPERIMENTS

Following a preliminary research, the task of the inventors was to develop a technology for decomposing cellulose acetate which had the advantage of producing mainly acetic acid and diethyl phthalate, focusing on hydrothermal liquefaction.

Hydrothermal liquefaction is a thermochemical process which occurs at medium temperature (about 270-330 °C) and at high pressure (5-15 MPa) in the presence of water. During the process, the water is in a subcritical state and acts as a solvent and reagent. The decomposition mechanism of cellulose acetate is hydrolysis starting from the ester bonds of the side chain which bind acetic acid to cellulose and then proceeding with the hydrolysis of the cellulose polymer.

According to the invention, the following treatment was developed:

HYDROTHERMAL TREATMENT:

In the typical route, 1 g of cellulose acetate and 5 g of water were fed into a micro-reactor. After having sealed it, the reactor was brought to operating temperature (190-250 °C) by means of a preheated sand bath. The reactor was maintained at that temperature for a constant time (10-240 min), after which it was extracted and cooled with cold water to room temperature.

After filtration, the aqueous phase was analyzed by means of GC-MS and GC-FID.

The solid residue was washed several times with distilled water, then oven dried at 80°C for 12h and weighed.

The equation for calculating the degradation rate was as follows:

$$\text{Degradation rate} = \left( M_{\text{cellulose acetate}} - M_{\text{solid residue}} / M_{\text{cellulose acetate}} \right)$$

The description will be better followed by referring to the accompanying drawings, in which:

Fig 1 shows the trend of the degradation rate of cellulose acetate under hydrothermal conditions at different temperatures and reaction times;

fig. 2 shows the degradation rate of the hydrothermal treatment with cellulose acetate with acidic and basic catalysts at 190°C;

fig. 3 shows the chemical composition of the aqueous phase obtained from the hydrothermal treatment of cellulose acetate;

**EXPERIMENTAL RESULTS**

OF THE HYDROTHERMAL TREATMENT OF CELLULOSE ACETATE

The first investigation carried out was to analyze the degradation rate of cellulose acetate under hydrothermal conditions at different temperatures and reaction times.

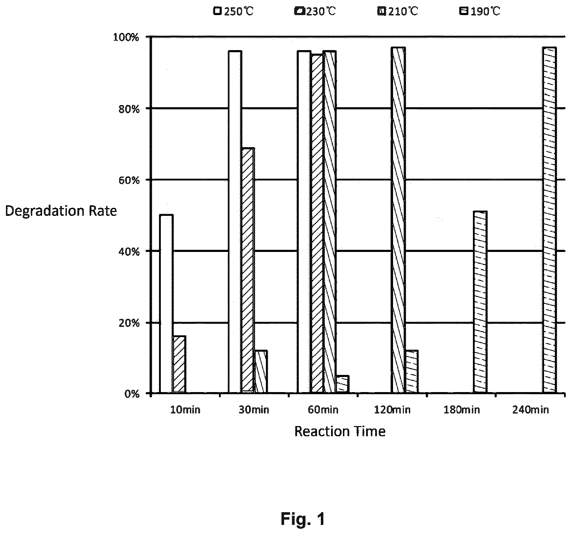

As shown in Fig. 1, higher temperature and reaction time are favorable to the degradation of cellulose acetate and the conditions required for an almost complete decomposition are: 250°C 30 min; 230°C 60 min; 210°C 60 min; and 190°C 240 min. At 190°C, higher reaction times (240 min) are needed to have a complete decomposition of the polymer, while at 250°C, only 30 min are needed.

So as to accelerate the cellulose acetate hydrolysis process, acidic and basic catalysts were used.

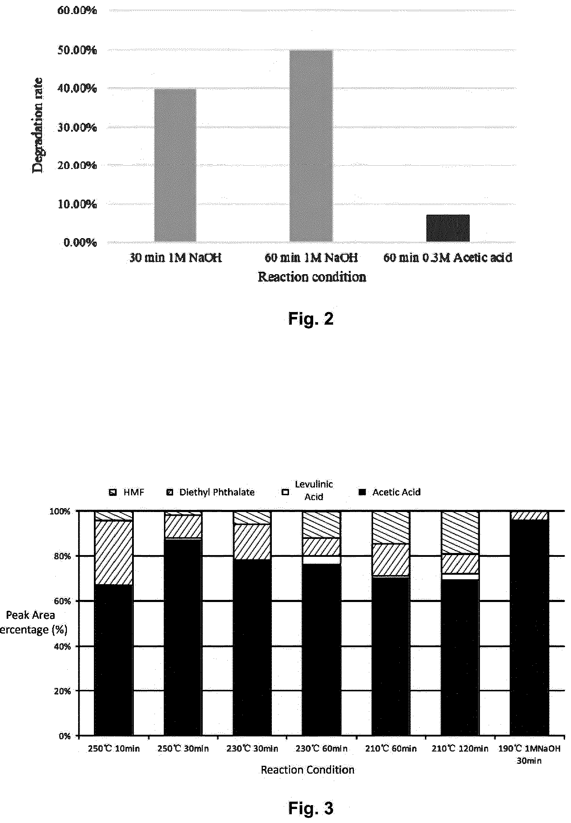

Fig. 2 shows the degradation rate of hydrothermal treatment with cellulose acetate with acidic and basic catalysts at 190 °C.

As shown in said Fig. 2, using 1 M NaOH solution where NaOH is an example of basic catalyst, after reaction at 190 °C for 60 min, 50% cellulose acetate was decomposed. Acidic catalysis appears not to be active in this process, indeed small amounts of acetic acid were added to the aqueous solution to check whether the reaction can be autocatalytic. An amount of 0.3 M acetic acid solution does not seem to produce any positive effect.

This can be explained by considering that acetic acid is the hydrolysis product of cellulose acetate, and the high concentration of acetic acid can promote the reverse reaction. NaOH significantly increased the degradation rate of cellulose acetate from 6% of the blank to 50% (190 °C for 60 min).

CHARACTERIZATION OF THE LIQUID PRODUCT

The aqueous phase products were analyzed by means of GC-MS (Gas Chromatography-Mass Spectrometry) and GC-FID (Gas Chromatography-Flame Ionization Detector). The main compounds are acetic acid, levulinic acid, diethyl phthalate and HMF, and the percentages related to the peak area are shown in Fig 3.

Diethyl phthalate is the plasticizer used in the production of cellulose acetate. Acetic acid could be produced by the hydrolysis of the acetyl group and also by the decomposition of the cellulose structure. Levulinic acid and HMF are the typical products of the hydrothermal liquefaction of cellulose in an acidic environment. The possible reaction route of the hydrothermal degradation of cellulose was proposed by Sudong Yin and Zhongchao Tan [I]. The cellulose was first hydrolyzed in monosaccharide (glucose), then converted into 5-HMF and finally transformed into levulinic acid and formic acid. The same phenomenon was found in the Applicant's work: together with the progress of the reaction, HMF was generated first, then levulinic acid was observed. Sudong Yin and Zhongchao Tan found that in an acidic reaction environment (pH=3, using HCl), the main products of the hydrothermal liquefaction of cellulose (300 °C, 0 min), are HMF (73.7%), acetic acid (17.9%) and levulinic acid (6.7%).

The quantification analysis of the aqueous phase products is carried out on GC-FID. The concentration of acetic acid in the aqueous phase product and the total molar amounts are reported in Table 2. Since the molecular weight of the repeating unit of cellulose acetate depends on the degree of substitution, as in the formula:

$$\text{Mr (g/mol)} = 159 + 43 \times DS + (3-DS) \times 1$$

For a degree of substitution of 2.45 it is 265 g/mol, the complete hydrolysis of the acetyl group of 1 g of pure cellulose acetate should lead to 0.00924 mol of acetic acid, meaning 0.55 $g/g_{cA}$. The acetic acid recovered from the hydrothermal process is calculated as: Acetic acid produced in the process/Amount of acetic acid in 1g of cellulose.

Table 2 : Concentration of acetic acid in the aqueous phase products.

| | 250 °C | | 230 °C | | 210 °C | | 190 °C 1M NaOH |
|---|---|---|---|---|---|---|---|
| | 10 min | 30 min | 30 min | 60 min | 60 min | 120 mins | 30 min |
| Concentration Acetic acid (g/mL) | 0.0168 | 0.0722 | 0.0285 | 0.0695 | 0.0499 | 0.0713 | 0.0268 |
| Recovered acetic acid % | 15.1 | 65.0 | 25.6 | 63.1 | 45.3 | 64. I | 24. |

CONCLUSIONS

From the experimentation conducted on cellulose acetate, it can be concluded that the hydrothermal liquefaction process can be used for recovering acetic acid from cellulose acetate waste and scrap, useful for the production of acetic anhydride and other valuable materials, by virtue of the high recovery rate thereof.

On the basis of the aforesaid laboratory results, an industrial process for recovering acetic acid and other valuable materials from cellulose acetate waste has thus been developed, where said cellulose acetate waste is subjected to a hydrothermal process operating at a temperature between 190 and 250 °C with reaction times between 10 and 240 min, feeding the process reactor with a feed of cellulose acetate and water having a weight ratio between 1:5 and 1:10.

The liquid phase is first separated from the residual solid, for example by decantation or filtration, and then subjected to a recovery process of acetic acid and diethyl phthalate including a first solvent extraction through which all the organic components are extracted and then a distillation to separate the different components from each other.

The acetic acid thus recovered can be sent to a plant for the traditional production of cellulose acetate, while advantageously the recovered diethyl phthalate can be mixed with the cellulose acetate produced, thus lowering the consumption of total diethyl phthalate.

The liquid fraction remaining from the recovery of acetic acid and diethyl phthalate can instead be treated to recover high added value compounds such as levulinic acid and HMF, thus increasing the total recovery of the plant.

**Claims**

1.  A process for recovering acetic acid and other valuable materials from cellulose acetate waste, **characterized in that** it includes:

    a) subjecting the cellulose acetate waste to a hydrothermal process operating at high pressure between 5 and 15 Mpa , at a temperature between 190 and 250 °C with reaction times between 10 and 240 min, feeding the reactor with a feed of cellulose acetate and water having a weight ratio between 1:5 and 1:10, in presence of acidic and basic catalysts;
    b) separating the liquid phase from the residual solid;
    c) sending the separated liquid phase to a process for recovering acetic acid and diethyl phthalate; and
    d) sending the recovered acetic acid to the plant for the traditional production of cellulose acetate.

2.  A process for recovering acetic acid and other valuable materials from waste according to claim 1, **characterized in that** the separation of the liquid phase from the residual solid occurs by decantation or filtration.

3.  A process for recovering acetic acid and other valuable materials from cellulose acetate waste according to claim 1, **characterized in that** the process of recovering acetic acid and diethyl phthalate from the liquid phase separated in b) occurs in two steps which include an extraction column with solvent followed by distillation.

4.  A process for recovering acetic acid and other valuable materials from cellulose acetate waste according to claim 1 **characterized in that** the recovered diethyl phthalate is mixed with the produced cellulose acetate, thus lowering the consumption of total diethyl phthalate.

5.  A process for recovering acetic acid and other valuable materials from waste according to claim 1, **characterized in that** the liquid fraction remaining from the recovery of acetic acid and diethyl phthalate is treated to recover high added value compounds such as levulinic acid and HMF, thus increasing the total recovery of the plant.

6.  A process according to claim 1, **characterized in that** higher temperatures and longer reaction times promote the degradation of cellulose acetate, whereby the conditions required for an almost complete degradation are: 250°C 30 min., 230°C 60 min, 210 °C 60 min and 190 °C 240 min.

7.  A process according to claim 1, **characterized in that** for temperatures of 250 °C and reaction times of 30 min, the recovery of acetic acid is greater than 60%.

8.  A process according to claim 1, **characterized in that** basic catalysts are usable to accelerate the hydrolysis process.

9.  A process according to claim 1, **characterized in that** the addition of an alkaline catalyst significantly increases the degradation rate of cellulose acetate from 6% of blank test to 50%, with 190 °C for 60 min.

10. A process according to claim 1, **characterized in that** the alkali catalyst is NaOH.

11. A process according to claim 1, **characterized in that** in an acidic pH environment, the hydrothermal liquefaction of cellulose at 250 °C leads to the production of 73.7% HMF (hydroxy methyl furfuran), 17.9% acetic acid, and 6.7% levulinic acid.

**Patentansprüche**

1.  Verfahren zur Gewinnung von Essigsäure und anderen Wertstoffen aus Celluloseacetatabfällen, **dadurch gekennzeichnet, dass** es umfasst:

    a) Unterziehen des Celluloseacetatabfalls einem hydrothermalen Verfahren bei hohem Druck zwischen 5 und 15 MPa, bei einer Temperatur zwischen 190 und 250 °C mit Reaktionszeiten zwischen 10 und 240 min, wobei der Reaktor mit einer Zufuhr aus Celluloseacetat und Wasser in einem Gewichtsverhältnis zwischen 1:5 und 1:10 in Gegenwart von sauren und basischen Katalysatoren gespeist wird;
    b) Abtrennung der flüssigen Phase vom restlichen Feststoff;
    c) Weiterleiten der abgetrennten flüssigen Phase zu einem Verfahren zur Gewinnung von Essigsäure und Diethylphthalat; und
    d) Weiterleiten der gewonnenen Essigsäure an die Anlage zur herkömmlichen Herstellung von Celluloseacetat.

2.  Verfahren zur Gewinnung von Essigsäure und anderen Wertstoffen aus Abfällen nach Anspruch 1, **dadurch gekennzeichnet, dass** die Trennung der flüssigen Phase vom festen Rest durch Dekantieren oder Filtrieren erfolgt.

3.  Verfahren zur Gewinnung von Essigsäure und anderen Wertstoffen aus Celluloseacetatabfällen nach Anspruch 1, **dadurch gekennzeichnet, dass** das Verfahren zur Gewinnung von Essigsäure und Diethylphthalat aus der in b) abgetrennten flüssigen Phase in zwei Schritten erfolgt, die eine Extraktionskolonne mit Lösungsmittel und eine anschließende Destillation umfassen.

4.  Verfahren zur Gewinnung von Essigsäure und anderen Wertstoffen aus Celluloseacetatabfällen nach Anspruch 1, **dadurch gekennzeichnet, dass** das gewonnene Diethylphthalat mit dem hergestellten Celluloseacetat gemischt wird, wodurch der Verbrauch an Gesamtdiethylphthalat gesenkt wird.

5.  Verfahren zur Gewinnung von Essigsäure und anderen Wertstoffen aus Abfällen nach Anspruch 1, **dadurch gekennzeichnet, dass** die bei der Gewinnung von Essigsäure und Diethylphthalat verbleibende flüssige Fraktion behandelt wird, um Verbindungen mit hohem Mehrwert, wie Lävulinsäure und HMF, zu gewinnen, wodurch die Gesamtgewinnung der Anlage erhöht wird.

6.  Verfahren nach Anspruch 1, **dadurch gekennzeichnet, dass** höhere Temperaturen und längere Reaktionszeiten den Abbau von Celluloseacetat fördern, wobei die für einen nahezu vollständigen Abbau erforderlichen Bedingungen sind: 250°C 30 Min., 230°C 60 Min., 210°C 60 Min. und 190°C 240 Min.

7.  Verfahren nach Anspruch 1, **dadurch gekennzeichnet, dass** bei Temperaturen von 250 °C und Reaktionszeiten von 30 min die Gewinnung von Essigsäure größer als 60 % ist.

8.  Verfahren nach Anspruch 1, **dadurch gekennzeichnet, dass** basische Katalysatoren zur Beschleunigung des Hydrolyseprozesses verwendet werden können.

9.  Verfahren nach Anspruch 1, **dadurch gekennzeichnet, dass** die Zugabe eines alkalischen Katalysators die Abbaugeschwindigkeit von Celluloseacetat von 6% der Blinprobe auf 50% bei 190°C für 60 min deutlich erhöht.

**10.** Verfahren nach Anspruch 1, **dadurch gekennzeichnet, dass** der alkalische Katalysator NaOH ist.

**11.** Verfahren nach Anspruch 1, **dadurch gekennzeichnet, dass** die hydrothermale Verflüssigung von Cellulose bei 250°C in einer sauren pH-Umgebung zur Herstellung von 73,7% HMF (Hydroxymethylfurfuran), 17,9% Essigsäure und 6,7% Lävulinsäure führt.

**Revendications**

**1.** Procédé de récupération d'acide acétique et d'autres substances de valeur à partir de déchets d'acétate de cellulose, **caractérisé en ce qu'**il comprend :

a) la soumission des déchets d'acétate de cellulose à un procédé hydrothermique mis en œuvre à haute pression entre 5 et 15 Mpa, à une température entre 190 et 250 °C avec des temps de réaction entre 10 et 240 min, en introduisant dans le réacteur une charge d'alimentation d'acétate de cellulose et d'eau présentant un rapport pondéral entre 1:5 et 1:10, en présence de catalyseurs acides et basiques ;
b) la séparation de la phase liquide du solide résiduel ;
c) l'envoi de la phase liquide séparée vers un procédé de récupération d'acide acétique et de phtalate de diéthyle ; et
d) l'envoi de l'acide acétique récupéré vers l'installation de production traditionnelle d'acétate de cellulose.

**2.** Procédé de récupération d'acide acétique et d'autres substances de valeur à partir de déchets selon la revendication 1, **caractérisé en ce que** la séparation de la phase liquide du solide résiduel a lieu par décantation ou filtration.

**3.** Procédé de récupération d'acide acétique et d'autres substances de valeur à partir de déchets d'acétate de cellulose selon la revendication 1, **caractérisé en ce que** le procédé de récupération d'acide acétique et de phtalate de diéthyle à partir de la phase liquide séparée en b) se déroule en deux étapes qui comprennent une colonne d'extraction avec solvant suivie d'une distillation.

**4.** Procédé de récupération d'acide acétique et d'autres substances de valeur à partir de déchets d'acétate de cellulose selon la revendication 1, **caractérisé en ce que** le phtalate de diéthyle récupéré est mélangé avec l'acétate de cellulose produit, ce qui permet de réduire la consommation en phtalate de diéthyle total.

**5.** Procédé de récupération d'acide acétique et d'autres substances de valeur à partir de déchets selon la revendication 1, **caractérisé en ce que** la fraction liquide qui reste après la récupération de l'acide acétique et du phtalate de diéthyle est traitée pour récupérer des composés à haute valeur ajoutée, tels que l'acide lévulinique et le HMF, ce qui augmente la récupération totale de l'installation.

**6.** Procédé selon la revendication 1, **caractérisé en ce que** des températures plus élevées et des temps de réaction plus longs favorisent la dégradation de l'acétate de cellulose, les conditions requises pour une dégradation presque complète étant : 250 °C 30 min, 230 °C 60 min, 210 °C 60 min et 190 °C 240 min.

**7.** Procédé selon la revendication 1, **caractérisé en ce que** pour des températures de 250 °C et des temps de réaction de 30 min, la récupération de l'acide acétique est supérieure à 60 %.

**8.** Procédé selon la revendication 1, **caractérisé en ce que** les catalyseurs basiques sont utilisables pour accélérer le procédé d'hydrolyse.

**9.** Procédé selon la revendication 1, **caractérisé en ce que** l'ajout d'un catalyseur alcalin augmente significativement le taux de dégradation de l'acétate de cellulose de 6 % de l'essai à blanc à 50 %, à 190 °C pendant 60 min.

**10.** Procédé selon la revendication 1, **caractérisé en ce que** le catalyseur alcalin est NaOH.

**11.** Procédé selon la revendication 1, **caractérisé en ce que** dans un environnement à pH acide, la liquéfaction hydrothermique de la cellulose à 250 °C conduit à la production de 73,7 % de HMF (hydroxyméthylfurfurane), 17,9 % d'acide acétique et 6,7 % d'acide lévulinique.

Fig. 1

**Fig. 2**

**Fig. 3**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 112047827 A